## Europäisches Patentamt
## European Patent Office
## Office européen des brevets

(19)

(11) Numéro de publication: **0 232 729**
**B1**

(12)

# FASCICULE DE BREVET EUROPEEN

(45) Date de publication du fascicule du brevet: **08.08.90**

(51) Int. Cl.⁵: **D 04 H 1/46,** D 04 H 1/42 // A61F13/15, A61F13/46

(21) Numéro de dépôt: **87100335.6**

(22) Date de dépôt: **13.01.87**

(54) Matelas fibreux absorbant, notamment pour des articles d'hygiène.

(30) Priorité: **15.01.86 FR 8600522**

(43) Date de publication de la demande:
**19.08.87 Bulletin 87/34**

(45) Mention de la délivrance du brevet:
**08.08.90 Bulletin 90/32**

(84) Etats contractants désignés:
**AT BE CH DE ES FR GB GR IT LI LU NL SE**

(56) Documents cités:
**EP-A-0 108 637**
**CH-A- 629 137**
**DE-B-1 635 682**
**FR-A-1 576 126**
**US-A-4 102 340**

(73) Titulaire: **PEAUDOUCE**
**59, Rue de la Vignette**
**F-59126 Linselles (FR)**

(72) Inventeur: **Koczab, Jean-Pierre**
**253 Domaine de la Vigne**
**F-59910 Bondues (FR)**

(74) Mandataire: **Casalonga, Axel et al**
**BUREAU D.A. CASALONGA - JOSSE**
**Morassistrasse 8**
**D-8000 München 5 (DE)**

Courier Press, Leamington Spa, England.

## Description

La présente invention se rapporte à un matelas absorbant à base de matériau fibreux, notamment pour des articles d'hygiène tels que des couches, des couches-culottes, des garnitures et des articles analogues.

Les matelas absorbants utilisés pour différents articles d'hygiène tels que couches, couches-culottes, garnitures pour incontinents, etc., sont constitués principalement par des matériaux absorbants fibreux, par exemple des fibres de cellulose (voir par exemple DE—B—1635682). Ces matériaux fibreux se présentent généralement sous la forme de "fluff" de cellulose, c'est-à-dire de fibres de cellulose très courtes (longueur de l'ordre de 1 à 2 mm) obtenues par défibrage à sec de pâte de papier. L'inconvénient principal de ces matériaux à base de fibres courtes réside dans le fait qu'à l'état mouillé, ils se défond, c'est-à-dire perdent leur cohésion, et s'agglomèrent ensuite sous la forme de boules.

On a également déjà proposé, en vue d'améliorer la capacité d'absorption de tels matelas, d'y incorporer des particules de matière dite superabsorbante. De tels superabsorbants sont généralement constitués par des polymères insolubles dans l'eau capables d'absorber plusieurs fois (au moins quinze fois) leur poids en liquide (voir par exemple EP—A—0 108 637).

Lorsque les particules de superabsorbant absorbent du liquide, elles gonflent et se transforment en une masse gélatineuse. Ces particules gélatineuses, lorsqu'elles sont proches les unes des autres, ont tendance à s'agglomérer et peuvent alors former une couche empêchant le passage ultérieur de liquide.

De très nombreuses propositions ont déjà été faites pour tenter d'éviter cette agglomération des particules de superabsorbant gonflées, gélatineuses, mais elles nécessitent pour la plupart des opérations supplémentaires (compression, embossage, traitement thermique, etc.) sur la ou les nappe(s) servant de support aux particules de superabsorbant, ce qui entraîne une augmentation du prix de revient. Par ailleurs, les traitements thermiques provoquent généralement une diminution de la capacité d'absorption des particules de superabsorbant, d'où une efficacité réduite du produit final.

La présente invention a pour objet un matelas absorbant à base de matériau fibreux, de structure simple, d'un prix de revient réduit, présentant une bonne cohésion, non seulement à l'état sec, mais également à l'état mouillé.

L'invention a également pour objet un matelas absorbant assurant une diffusion rapide du liquide à travers l'ensemble du matelas.

L'invention a par ailleurs pour objet un matelas absorbant pouvant contenir des particules de superabsorbant dans lequel les particules de superabsorbant sont bien retenues en position et ne risquent pas de s'agglomérer à l'état gonflé.

Le matelas absorbant conforme à l'invention à base de matériau fibreux, notamment pour des articles d'hygiène tels que des couches, des couches-culottes, des garnitures, comprend au moins une nappe de fibres d'une longueur au moins égale à l'épaisseur de la nappe, la nappe étant liée par aiguilletage ou un procédé analogue utilisant les fibres de la nappe pour le liage de cette dernière.

C'est grâce à l'utilisation de fibres longues au lieu de fibres courtes pour la constitution du matelas absorbant qu'il est possible de lier ce dernier par aiguilletage ou un procédé analogue et de donner ainsi au matelas une bonne cohésion que le matelas conserve également à l'état mouillé.

Le matelas absorbant peut, par exemple, être constitué par une unique nappe aiguilletée de fibres, mais peut avantageusement comprendre au moins deux nappes formées chacune de fibres longues, les deux nappes étant liées par aiguilletage ou un procédé analogue.

Dans ce cas, il peut être avantageux de former au moins l'une des nappes, par exemple celle qui sera éloignée du corps de l'utilisateur de l'article d'hygiène par des fibres absorbantes et de former une autre nappe, par exemple celle qui sera tournée vers le corps de l'utilisateur à partir de fibres non absorbantes.

L'avantage particulier d'une telle structure de matelas absorbant réside dans le fait que des fibres absorbantes d'au moins une nappe sont aiguilletées à travers la nappe de fibres non absorbante et forment ainsi, à travers cette dernière, des drains favorisant le passage du liquide à travers la nappe de fibres non absorbante vers la ou les nappes de fibres absorbantes.

Suivant un mode de réalisation préféré, le matelas absorbant comprend, en outre, des particules de superabsorbant entre au moins deux nappes de fibres longues liées par aiguilletage ou un procédé analogue.

Cela permet non seulement de réduire considérablement l'épaisseur du matelas absorbant pour une même capacité d'absorption, mais encore d'obtenir, par l'aiguilletage des nappes recevant entre elles les particules de superabsorbant, un emprisonnement des particules de superabsorbant entre les points d'aiguilletage, empêchant ou pour le moins réduisant la tendance des particules de superabsorbant à s'agglomérer à l'état gonflé.

Chacune des nappes de fibres longues du matelas conforme à l'invention peut être préaiguilletée sur elle-même avant que les nappes réunies soient aiguilletées ensemble.

Le matelas conforme à l'invention peut également comprendre, pour améliorer la diffusion du liquide sur l'ensemble du matelas, une feuille de ouate de cellulose entre les nappes liées par aiguilletage.

Par ailleurs, il est possible de munir le matelas conforme à l'invention d'une feuille de ouate de cellulose ou de non-tissé sur au moins une face extérieure du matelas, en vue de la consolidation et de l'amélioration du confort du matelas.

En se référant aux dessins schématiques annexés, on va décrire ci-après plus en détail, plusieurs modes de réalisation illustratifs et non limitatifs d'un matelas absorbant conforme à l'invention; sur les dessins:

la figure 1 est une coupe schématique partielle d'un matelas absorbant comprenant une nappe de fibres longues absorbantes doublée sur les deux faces d'une couche de ouate;

la figure 2 est une coupe schématique partielle d'un matelas absorbant comprenant deux nappes de fibres longues, l'une à fibres absorbantes et l'autre à fibres non absorbantes, doublées extérieurement, l'une d'une couche de ouate de cellulose et l'autre d'un non-tissé, des grains de superabsorbant étant disposés entre les deux nappes.

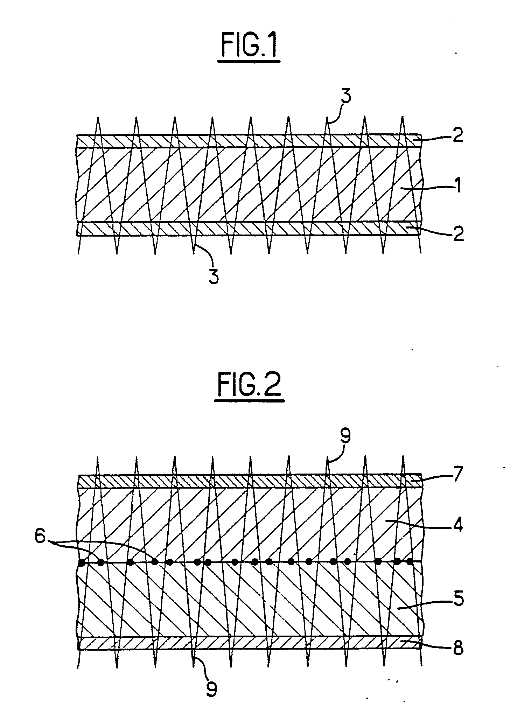

Selon la figure 1, un matelas absorbant est formé d'une nappe 1 de fibres absorbantes longues, d'une longueur supérieure à l'épaisseur de la nappe 1. Les fibres de la nappe 1 peuvent être, par exemple, des fibres de viscose ou encore des fibres acryliques à structure microporeuse telles que les fibres commercialisées sous la marque "Dunova" par la Société BAYER, R.F.A.

La nappe de fibres 1 est doublée sur chaque face d'une couche de ouate de cellulose 2.

La nappe 1 doublée par les couches de ouate 2 est aiguilletée depuis les deux faces, comme indiqué schématiquement par les lignes 3, de telle manière que les fibres aiguilletées traversent également les deux couches de ouate 2. Cet aiguilletage assure donc à la fois une bonne cohérence à la nappe de fibres 1, et la liaison des couches de ouate 2 avec la nappe de fibres 1.

Selon la figure 2, un matelas absorbant comprend deux nappes 4, 5 de fibres longues, la nappe 4 pouvant être constituée par des fibres non absorbantes, par exemple des fibres de polyester, et la nappe 5 par des fibres absorbantes, par exemple des fibres de viscose ou des fibres acryliques à structure microporeuse.

Entre les deux nappes 4, 5, sont disposées des particules 6 de matière superabsorbante du commerce, par exemple des polymères à base d'acide polyacrylique ou des dérivés d'amidon ou de cellulose obtenus par greffage, par exemple polysaccharide, alginate, carboxyméthylcellulose, sous forme de poudres, fibres, laminés, etc. De telles matières superabsorbantes sont par exemple commercialisées sous la marque "Lucquasorb" de la Société BASF, R.F.A., ou sous la marque "Aquakeep" de la Société SEITETSU KAGAKU, JAPON.

La nappe 4 de fibres non absorbantes est doublée extérieurement d'une couche 7 de non-tissé, par exemple de viscose ou de polyester, et la nappe 5 de fibres absorbantes est doublée extérieurement d'une couche 8 de ouate de cellulose.

Les deux nappes 4, 5, la couche 7 de non-tissé et la couche 8 de ouate de cellulose, sont liées par aiguilletage de l'ensemble du matelas composite, depuis les deux faces, les fibres des deux nappes 4, 5 étant aiguilletées de manière à traverser les nappes 5, 4 et les couches 7, 8, respectivement, comme indiqué schématiquement par les lignes 9.

Cet aiguilletage par les fibres longues des nappes 4 et 5 procure non seulement une bonne cohésion à chaqune des deux nappes 4, 5, mais assure également la liaison des nappes 4, 5 avec la couche 7 de non-tissé et la couche 8 de ouate. Par ailleurs, cet aiguilletage assure le positionnement des particules de superabsorbant 6, c'est-à-dire que les particules 6 sont emprisonnées entre les points d'aiguilletage, de manière que leur gonflement ne soit pas gêné, mais que les particules gonflées ne puissent pas s'agglomérer.

Enfin, les fibres absorbantes de la nappe 5, aiguilletées à travers la nappe 4 de fibres non absorbantes et le non-tissé 7 forment des drains qui favorisent le passage, à travers le non-tissé 7 et la nappe 4 de fibres non absorbantes, du liquide arrivant sur le non-tissé 7, vers la nappe 5 qui assure la dispersion du liquide sur tout le matelas ainsi que l'absorption du liquide, en combinaison avec les particules 6 de superabsorbant.

Du point de vue fabrication, il peut éventuellement être préférable de soumettre chaque nappe 4, 5 à un préaiguilletage avec la couche 7 ou 8 associée et de soumettre ensuite les deux structures composites partielles ainsi formées à un aiguilletage portant sur l'ensemble du matelas composite ainsi constitué.

Il va de soi que les modes de réalisation décrits ci-dessus ne sont donnés qu'à titre d'exemples illustratifs et non limitatifs et que de nombreuses modifications et variantes sont possibles dans le cadre de l'invention.

Ainsi, sur un matelas formé de deux nappes de fibres longues, avec particules de superabsorbant entre les deux nappes, ces deux nappes pourraient être constituées par des fibres absorbantes, identiques ou différentes, ou par des fibres non absorbantes, identiques ou différentes, ou par des mélanges de fibres absorbantes et de fibres non absorbantes.

Il est également possible d'incorporer à un matelas absorbant conforme à l'invention, comprenant par exemple deux nappes de fibres longues, au moins une épaisseur de fibres courtes, par exemple de fibres de cellulose (fluff), dans la mesure où l'aiguilletage de l'ensemble du matelas par les fibres des nappes à fibres longues permet également de lier simultanément, donc de consolider, la couche de "fluff". Cette couche de "fluff" est alors de préférence située entre les deux couches de fibres longues, de même qu'une ou plusieurs feuilles de ouate de cellulose peuvent être disposées entre les deux nappes de fibres longues, notamment pour améliorer la diffusion du liquide sur l'ensemble du matelas.

Il s'est avéré avantageux d'utiliser des fibres longues ayant, selon l'épaisseur totale du matelas, une longueur comprise entre environ 20 et 80 mm et un titre compris entre environ 0,75 et 6 deniers.

Les particules de superabsorbant, sous forme de grains, de fibres ou autres, peuvent être utilisées de préférence à raison d'environ 50 à 150 g/m² de matelas.

Enfin, au lieu de procéder à un aiguilletage classique, il serait également possible d'utiliser des procédés analogues de liage utilisant les fibres des nappes à lier, par exemple le procédé connu sous le non de "couture-tricotage", mis en oeuvre sans apport de fil.

## Revendications

1. Matelas absorbant à base de matériaux fibreux, notamment pour des articles d'hygiène tels que des couches, des couches-culottes, des garnitures, caractérisé par le fait qu'il comprend au moins une nappe (1; 4, 5) de fibres d'une longueur au moins égale à l'épaisseur de la nappe, la nappe étant liée par aiguilletage ou un procédé analogue faisant appel aux fibres de la nappe elle-même pour le liage de cette dernière.

2. Matelas suivant la revendication 1, caractérisé par le fait qu'il comprend au moins deux nappes (4, 5) formées chacune de fibres d'une longueur au moins égale à l'épaisseur de la nappe, et que les deux nappes sont liées entre elles par aiguilletage ou un procédé analogue.

3. Matelas suivant la revendication 2, caractérisé par le fait que l'une au moins des deux nappes est formée de fibres absorbantes.

4. Matelas suivant la revendication 3, caractérisé par le fait qu'une autre nappe au moins est formée de fibres non absorbantes.

5. Matelas suivant l'une quelconque des revendications 2 à 4, caractérisé par le fait qu'il comprend, en outre, des particules de superabsorbant entre deux nappes.

6. Matelas suivant l'une quelconque des revendications 2 à 5, caractérisé par le fait que chaque nappe est préaiguilletée en elle-même et que les nappes sont aiguilletées ensemble.

7. Matelas suivant l'une quelconque des revendications précédentes, caractérisé par le fait qu'il comprend, en outre, une feuille (2, 8) de ouate de cellulose sur une face d'au moins une nappe de fibres longues.

8. Matelas suivant l'une quelconque des revendications précédentes, caractérisé par le fait qu'il comprend, en outre, une feuille (7) de non-tissé sur une face d'au moins une nappe de fibres longues.

## Patentansprüche

1. Saugfähiges Kissen aus Faserwerkstoff, insbesondere für Hygieneprodukte, wie Windeln, Windelhöschen, Damenbinden, dadurch gekennzeichnet, daß es wenigstens eine Matte (1; 4, 5) aus Fasern mit einer Länge wenigstens gleich der Dicke der Matte aufweist, wobei die Matte durch Nadeln oder ein ähnliches Verfahren gebunden ist, bei dem die eigenen Fasern der Matte zu deren Bildung benutzt werden.

2. Kissen nach Anspruch 1, dadurch gekennzeichnet, daß es wenigstens zwei Matten (4, 5) aufweist, die jede aus Fasern mit einer Länge wenigstens gleich der Dicke der Matte gebildet sind, und daß die beiden Matten miteinander durch Nadeln oder ein ähnliches Verfahren gebunden sind.

3. Kissen nach Anspruch 2, dadurch gekennzeichnet, daß wenigstens eine der beiden Matten von saugfähigen Fasern gebildet ist.

4. Kissen nach Anspruch 3, dadurch gekennzeichnet, daß wenigstens eine andere Matte von nicht saugfähigen Fasern gebildet ist.

5. Kissen nach einem der Ansprüche 2 bis 4, dadurch gekennzeichnet, daß es weiters zwischen zwei Matten superabsorbierende Teilchen aufweist.

6. Kissen nach einem der Ansprüche 2 bis 5, dadurch gekennzeichnet, daß jede Matte in sich selbst vorgenadelt ist und daß die beiden Matten zusammengenadelt sind.

7. Kissen nach einem der vorhergehenden Ansprüche, dadurch gekennzeichnet, daß es weiters ein Blatt (2, 8) aus Zellstoffwatte auf einer Seite wenigstens einer Matte aus langen Fasern besitzt.

8. Kissen nach einem der vorhergehenden Ansprüche, dadurch gekennzeichnet, daß es weiters ein Vlies (7) auf einer Seite wenigstens einer Matte aus langen Fasern besitzt.

## Claims

1. Absorbent pad with a base of fibrous materials, in particular for hygiene articles such as nappies, nappy-pants and sanitary towels, characterized in that it comprises at least one web (1; 4, 5) of fibres which have a length equal to at least the thickness of the web, the web being bound by needling or a similar method making use of the fibres of the web itself to bind the latter.

2. Pad according to Claim 1, characterized in that it comprises at least two webs (4, 5) each formed from fibres which have a length equal to at least the thickeness of the web, and that the two webs are bound together by needling or a similar method.

3. Pad according to Claim 2, characterized in that at least one of the two webs is formed from absorbent fibres.

4. Pad according to Claim 3, characterized in that at least one other web is formed from nonabsorbent fibres.

5. Pad according to any one of Claims 2 to 4, characterized in that it furthermore comprises particles of superabsorbent material between two webs.

6. Pad according to any one of Claims 2 to 5, characterized in that each web is individually needled beforehand and that the webs are needled together.

7. Pad according to any one of the preceding claims, characterized in that it furthermore comprises a sheet (2, 8) of cellulose wadding on one face of at least one web of long fibres.

8. Pad according to any one of the preceding claims, characterized in that it furthermore comprises a sheet (7) of nonwoven on one face of at least one web of long fibres.

## FIG.1

## FIG.2

1